# EUROPEAN PATENT SPECIFICATION

(11) **EP 0 443 856 B1**
(45) Date of publication and mention of the grant of the patent: **26.04.1995**
(21) Application number: 91301397.5
(22) Date of filing: 21.02.1991
(51) Int. Cl.: C07C 63/331, C07C 51/265, C07D 307/89

(54) **Preparation of isopropylidene bis(phthalic acid) and isopropylidene bis (phthalic anhydride)**
Herstellung von Isopropylidenbisphthalsäure und von Isopropylidenbisphthalsäureanhydrid
Préparation de l'acide isopropylidène bis-phtalique et du bisanhydride de l'acide isopropylidène bis-phtalique

(30) Priority: 22.02.1990 US 484354; 22.02.1990 US 484346
(43) Date of publication of application: 28.08.1991
(73) Proprietor: AMOCO CORPORATION, Chicago Illinois 60601 (US)
(72) Inventor: Hussman, Gregory Paul, Batavia Illinois 60510 (US); Bleull, Anthony Dean, Crown Point, Indiana 46307 (US); Sanchez, Paul Anthony, Lisle, IL 60532 (US)
(74) Representative: Ritter, Stephen David

(56) References cited:
- US-A- 2 712 543
- US-A- 3 161 693
- PATENT ABSTRACTS OF JAPAN, unexamined applications, C section, vol. 13, No.156, April 14, 1989 THE PATENT OFFICE JAPANESE GOVERNMENT page 30 C 585

## Description

This invention relates to the preparation of isopropylidene bis(phthalic acid) and isopropylidene bis(phthalic anhydride).

### Background of Invention

Isopropylidene bis(phthalic acid), also known as 2,2-bis-(3,4-dicarboxyphenyl)propane (IBPA), is a useful chemical intermediate. For example, this tetra-carboxylic acid can be dehydrated to produce isopropylidene bis(phthalic anhydride) (IPAN) from which polymers with enhanced blending properties in polyether ketone formulations can be prepared. Alternatively, the dianhydride can be converted to an ester which can then be used in the manufacture of polyimide resin having a reduced dielectric constant and useful in electronic composites.

Oxidation processes for producing IBPA from dixylylpropane (DXP), also known as isopropylidene bis(xylene) and as 2,2-bis-(3,4-dimethylphenyl)propane, have previously been described in, for example, Gresham et al. U.S. Patent No. 2,712,543 where nitric acid and also potassium permanganate oxidation of DXP to IBPA are taught. However, both these oxidation processes consume relatively large amounts of inorganic reagents, involve elaborate procedures, and produce IBPA yields of questionable economic value, as well as a relatively large number of undesired by-products.

Furthermore, unlike most tetra-acids, IBPA is completely soluble in oxidation reactor effluent. As a result, isolation of a purified IBPA from such an oxidation by simple filtration for purposes of permitting a subsequent dehydration of purified IBPA to IPAN is not possible. To overcome this problem, Gresham et al. refluxed a mixture of the crude IBPA and xylene which partially dehydrated the crude IBPA into a crude IPAN. Such refluxed product was evaporated to dryness. Subsequent treatment with acetic anhydride to accomplish "complete" anhydride formation is described. Depending upon the method of the oxidation, the crude IPAN was finally purified by acetone extraction, by various washing procedures, and by multiple recrystallization procedures (from xylene). Because of the impurities produced by the indicated oxidation procedures, and also because of the multiple steps associated with the purification procedures, the Gresham et al. DXP preparation procedures are not practical for commercial production of IBPA or IPAN.

A possibly more promising oxidative route for preparing IBPA from DXP would be to utilize an alkyl aromatic oxidation process such as the so-called "Mid-Century Oxidation Process" generally described by Towle et al., "Make Most Aromatic Acids Using Mid-Century Oxidation Processes," in "Petrochemical Developments," November, 1964, Vol. 43, No. 11, pp. 149-152. See also Baldwin, U.S. Patent No. 3,064,044, Marsh et al., U.S. Patent No. 4,081,464, and Brown et al., U.S. Patent No. 4,587,355. However, liquid phase, elevated temperature oxidation of DXP using a cobalt/manganese/bromine catalyst as in the Mid-Century oxidation process is not very efficient for the manufacture of IBPA. Relatively low yields of IBPA are obtained, and unacceptably high amounts of an unwanted by-product, trimellitic acid, are produced.

A new and improved oxidation process for converting DXP to IBPA would be economically advantageous. The present invention provides such a process.

However, because of the above referenced solubility characteristics of IBPA, the problems of preparing a purified IBPA or a purified dehydrated IPAN from the IBPA containing product of such an alkyl aromatic oxidation process remain. Even removal of the oxidation solvent used in such process (aqueous aliphatic carboxylic acid) by distillation does not promote precipitation of IBPA, but rather results in the formation of an intractable oil.

The art needs a simple, effective, and economical technique for preparing purified IPAN from the crude oxidation reactor effluent containing the IPBA. The present invention satisfies that need.

### Summary of the Invention

Dixylylpropane is oxidized to isopropylidene bis(phthalic acid) in relatively high yields using a liquid phase oxidation process and an oxidation catalyst system that includes zirconium. As compared to prior art oxidation processes, in the present process the amount of by-products, especially of trimellitic acid (TMA), is substantially reduced.

The oxidation catalyst system contemplated by the present invention includes zirconium together with conventional catalysts for the liquid phase oxidation of alkyl aromatics. A preferred catalyst system includes forms of zirconium, cobalt, manganese and bromine which are soluble in the oxidation solvent employed.

Thus, the invention provides a method of producing isopropylidene bis(phthalic acid) which comprises oxidising dixylylpropane with a source of molecular oxygen in the liquid phase in the presence of a catalyst and an aqueous oxidation solvent comprising an aliphatic acid having 2 to 6 carbon atoms, characterised in that the catalyst comprises sources of zirconium, cobalt, manganese and bromine.

A surprising and unexpected feature of the present process is that the presence of relatively small amounts of zirconium drives the oxidation reaction towards completion and towards high yields of IBPA with minimal production of TMA, particularly when the process is practiced at relatively low temperatures and with relatively low molar ratios of bromine to the catalytically-active metals cobalt and manganese.

More specifically, the presently contemplated method for producing IBPA utilizes liquid phase oxidation of DXP and includes the steps of introducing into an oxidation reactor, or reaction zone, a DXP-containing admixture which is maintained in the reactor at an elevated temperature in the range of 100°C to 240°C (212°F to 464°F), and preferably at autogenous pressure, for a time period sufficient to convert at least some of the introduced DXP to IBPA. The reaction admixture includes, in addition to DXP, an oxidation solvent that contains a C₂ to C₆ aliphatic acid, an oxygen-containing gas, and an oxidation catalyst system containing zirconium and additionally constituted by cobalt, manganese, and bromine, all in forms soluble in the aforementioned oxidation solvent. The oxidation solvent can also contain up to 25 weight percent water on a total solvent basis, although the present particularly preferred oxidation solvent is a mixture comprised on a total solvent basis of 95 weight percent acetic acid and 5 weight percent water. An effluent stream having a reduced DXP content, but containing IBPA, is produced and is withdrawn from the reactor.

Either a batch or a continuous process scheme can be utilized in practicing the present oxidation process.

The present oxidation process can be practiced efficiently and economically. Advantageously, existing equipment for carrying out the well-known Mid-Century alkyl aromatic oxidation process can be used so that capital costs for producing IBPA from DXP can be minimized so far as equipment considerations are concerned.

The catalyst employed oxidizes DXP to IBPA at relatively high conversion rates with minimum by-product (especially TMA) production.

Additionally, relatively high purity isopropylidene bis(phthalic anhydride) (IPAN) is, in this invention, produced from a crude aqueous isopropylidene bis(phthalic acid) (IBPA) solution by dehydration of the dissolved IBPA in the presence of an aromatic hydrocarbon solvent for IPAN that also forms a minimum boiling azeotrope with water. The dehydration is effected at an elevated temperature, and, as water is generated, it is removed by azeotropic distillation. A purified acid anhydride is recovered as a precipitate from the heated solution upon cooling.

More particularly, this dehydration involves combining crude aqueous IBPA solution with the aromatic hydrocarbon solvent, and heating the resulting admixture to an elevated temperature that is sufficient to dehydrate the acid moiety. At the same time the corresponding dianhydride is dissolved in such solvent. The admixture is maintained at the dehydration temperature for a time period sufficient to effect the desired, substantially complete dehydration while the dehydration water is removed substantially concurrently by azeotropic distillation. A heated liquid residue containing dissolved IPAN in the hydrocarbon solvent is thus produced. This heated liquid residue may be filtered, if desired, to separate therefrom particulate insoluble impurities, and is then cooled to cause precipitation of solid IPAN of relatively high purity. The precipitate is recovered from the cooled liquid residue by liquid-solids separation.

The present dehydration process can produce purified IPAN directly from a crude oxidation reactor effluent containing IBPA in an efficient, simple, and low cost manner. Nothing in the prior art teaches or suggest a process for dehydrating IBPA to produce directly a precipitated, substantially pure IPAN without any intervening evaporation step, washing step, or solvent recrystallization step. The need for intermediate isolation of IBPA is eliminated, thus eliminating several steps normally required in the production and purification of dianhydrides. The relatively high purity IPAN product recovered also generally eliminates the need for any additional dehydration and purification.

Optionally, the water content of the starting reactor effluent can be first reduced by an initial distillation to remove most of the oxidation solvent, and the dehydration carried out thereafter.

Various other and further features, embodiments, and the like which are associated with the present invention will become apparent to those skilled in. the art from the present description. It is to be expressly understood, however, that the associated accompanying portions of this specification are provided for purposes of illustration and description only, and are not intended as limitations on the invention.

### Detailed Description of Preferred Embodiments

In the practice of the oxidation process of the present invention, DXP is heated in a reaction zone under liquid phase conditions and in the presence of an oxidation catalyst. The following materials are charged to the reaction zone:
- dixylylpropane,
- oxidation solvent containing at least one aliphatic carboxylic acid having from 2 to 6 carbon atoms per molecule,
- oxygen containing gas, such as air or oxygen, and
- zirconium containing oxidation catalyst.

The oxidation solvent can optionally contain up to 25 weight percent water on a total solvent weight basis. A present preference is to employ an oxidation composition comprised of a mixture of such an aliphatic acid and water wherein, on a 100 weight percent total composition basis, the quantity of aliphatic acid is in the range of 99.5 to 80 weight percent, and, correspondingly, the quantity of water is in the range of 0.5 to 20 weight percent. A present particularly preferred oxidation solvent is a mixture of 95 weight percent acetic acid and 5 weight percent water (total solvent weight basis).

The weight ratio of the oxidation solvent to the DXP in the reaction zone is preferably in the range of 2:1 to 10:1, and more preferably is in the range of 2.5:1 to 8:1. The amount of oxygen-containing gas charged to the reaction zone is preferably at least sufficient to achieve and maintain a molar excess of oxygen relative to the DXP present.

In addition to zirconium, the oxidation catalyst includes cobalt, manganese, and bromine components, all of which are soluble in the oxidation solvent. Preferably, these catalyst components are preliminary dissolved in the solvent (or a portion of the solvent) before being charged to the reaction zone. The respective amounts of each of the components is chosen so that there are present in the reaction zone 1 to 50 milligram atoms of cobalt calculated as elemental cobalt per gram mole of DXP or 0.05 to 3 milligram atoms of cobalt per 100 parts by weight of the solution, 0.1 to 10 milligram atoms of manganese calculated as elemental manganese per milligram atom of cobalt calculated as elemental cobalt, 0.01 to 1 milligram atoms of zirconium calculated as elemental zirconium per milligram atom of cobalt calculated as elemental cobalt, and bromine in a bromine-to-(cobalt plus manganese) respective mole ratio of 0.02 to 1.

The use of zirconium in combination with the other catalyst components is believed to drive the oxidation reaction of DXP to completion, and thus to provide high yields of IBPA with minimum TMA production.

Examples of suitable aliphatic acids which may be employed as solvents in the practice of the oxidation process of this invention include acetic acid (presently preferred), propionic acid, n-butyric acid, isobutyric acid, n-valeric acid, trimethylacetic acid, caproic acid, and the like. Water can be, and typically is, present in the solvent.

Any convenient source of molecular oxygen may be employed for the oxidation process of this invention. Air is presently preferred as such source. The oxygen content of the molecular oxygen source can vary, for example, from that of the oxygen content of atmospheric air up to that of industrial grade oxygen and above.

The above indicated combination of materials is maintained in the reaction zone with agitation at a temperature in the range of 100°C to 240°C (212°F to 464°F) and preferably 140°C to 215°C (284°F to 419°F) while pressures are maintained in the reaction zone which are at least sufficient to maintain liquid phase conditions. These pressures are typically and conveniently autogenous pressures. Suitable reactor gauge pressures typically are in the range of 10 kg/cm² to 30 kg/cm², and can be as high as 35 kg/cm². During the oxidation, the components in the reaction zone are stirred using an agitator means, such as conventional impellers, or the like. Such a temperature and such a pressure is preferably maintained for a time sufficient to oxidize at least 75 mole percent of the charged dixylylpropane.

The oxidation process of the present invention can be practiced batchwise or continuously.

Preferably, the residence time of the components in the reaction zone at the particular elevated temperatures and pressures employed is sufficient to accomplish a conversion of at least 85 weight percent of the DXP (based on charged weight of such starting compound). Typical and illustrative residence times needed to achieve such a conversion level are in the range of 15 to 60 minutes for inverse reaction temperatures in the range of 220°C to 250°C (428°F to 482°F) although longer and shorter times can be used, depending upon variables such as temperature, catalyst concentration conversion desired, throughput ratio, available process equipment, and the like.

The yield of IBPA is more preferably greater than 85 mole percent, based on starting DXP. In general, the amount of by-product TMA produced is characteristically less than 4 mole percent, and, more preferably, is kept at less than 2 mole percent, based on starting DXP.

The oxidation reaction of DXP to IBPA that is accomplished in the reaction zone proceeds relatively quickly. A substantial portion of the heat generated by the exothermic oxidation reaction is removed from the reaction mixture by vaporization of the solvent, and, to a smaller extent, of the DXP. The vaporized material and any unreacted oxygen and other gaseous components of, for example, an air feed to the reaction zone, pass preferably upwardly through the reaction zone and are withdrawn from the reaction zone above the level of the liquid reaction mixture in the reaction zone. The gaseous effluent from the reaction zone is passed through a reflux condenser, or the like, wherein the vaporized solvent and DXP are condensed for recycle or reuse. The non-condensable gases are conveniently vented.

Even under batch operating conditions, it is preferred to introduce oxygen-containing gas continuously into, and to withdraw gaseous effluent continuously from, an agitated reaction zone. This procedure is primarily done because it is desirable to maintain relatively high oxygen partial pressures in the reaction zone. Such excesses have the beneficial effect of reducing undesirable side reactions and also of favoring formation of the desired IBPA. It is presently more preferred that the oxygen-containing gas be fed to the reaction zone at a rate which results in an exhaust gas-vapor mixture containing 1.8 to 8 volume percent oxygen (measured on a solvent-free basis). Such an exhaust gas oxygen content is believed to be sufficient to provide in the reaction zone the desired level of oxygen concentration in the range of 1.6 to 2.8 moles of oxygen per methyl group of the DXP.

In general, a catalyst system used in the practice of the oxidation process of the present invention is provided by a combination of at least one solvent soluble zirconium compound together with solvent soluble catalyst components of the type heretofore conventionally used for liquid phase oxidation of alkyl aromatics, such as those comprised of a mixture of solvent soluble compounds of zirconium, cobalt, manganese and bromine.

For example, when a continuous oxidation process embodying this invention is contemplated, the feed stream(s) introduced into the reaction zone preferably contain(s) each of the DXP and catalyst system already dissolved in solvent. The weight ratio of the solvent containing dissolved catalyst and dissolved DXP fed into the reaction zone to the total amount of solvent introduced into the reaction zone is adjustable, but a present preference is to employ a ratio of solvent to dixylylpropane in the range of 2.5:1 to 8:1 in the reaction zone. If desired, the DXP and the catalyst can be introduced into the reaction zone separately from the solvent rather than being preliminarily dissolved in the solvent charged in a feed stream. A molar excess of oxygen relative to DXP is employed.

When the starting catalyst composition is comprised of dissolved forms of cobalt, manganese, zirconium, and bromine, it is presently preferred that the cobalt component (calculated as elemental cobalt,) be present in such solution at a level of 0.05 to 3 milligram atoms of cobalt per each 100 parts by weight of such solution. It is also preferred that the cobalt (calculated as elemental cobalt) be present in the range of 1 to 50 milligram atoms (mga) dissolved in the oxidation solvent per gram mole of the DXP; the manganese (calculated as elemental manganese) be present in the range of 0.1 to 10 mga dissolved in the oxidation solvent per milligram atom of cobalt (calculated as elemental cobalt); the zirconium (calculated as elemental zirconium) be present in the range from 0.01 to 1 mga dissolved in the oxidation solvent per milligram atom of cobalt; and the bromine (calculated as the elemental bromine ion) be present in the range of 0.05 to 2.5 mga dissolved in the oxidation solvent per milligram atom of cobalt. More preferably, and particularly in the case of practicing this invention with such a continuous process, these catalyst components are dissolved in the solvent in such respective indicated amounts with the mole ratio of bromine to total moles of cobalt and manganese present being in the range of 0.02 to 1.

When such a starting catalyst composition is being used in a system wherein the weight ratio of solvent employed to the amount of DXP charged is within the preferred range above indicated, the cobalt quantity present is preferably in the range of 0.003 to 0.17 weight percent (calculated as elemental cobalt) based on 100 weight percent combined cobalt and solvent with the respective amounts of manganese, zirconium, and bromine remaining as above defined in relation to cobalt.

In a preferred embodiment of the oxidation method of this invention, the preferred solvent is a mixture of acetic acid with water, such as above described, with each of the metals cobalt, manganese, and zirconium being provided in any of their known acetic acid-soluble ionic or combined forms. For example, the cobalt and manganese can each be introduced as the carbonate, the acetate tetrahydrate, and/or the bromide. The zirconium can likewise be introduced, if desired, as the carbonate, the acetate tetrahydrate, and/or the bromide. Zirconium can be added in any form that is soluble in the oxidation solvent. For example, ZrO₂ is commercially available from the Sheppard Chemical Company, Cincinnati, Ohio, as a solution in acetic acid, and such composition is very well suited for use in the practice of this invention.

Because of (1) the aforesaid requirements regarding the mole ratio of bromine to cobalt and manganese (each calculated as the elemental metal), and (2) the fact that the bromides of zirconium, cobalt and manganese inherently have a bromide to metal gram atom ratio of 2:1, the catalyst system used in this invention cannot be provided by the use of only bromides of zirconium, cobalt and manganese. Rather, the desired catalyst system is provided by selecting appropriate ratios of such bromide salts and other aliphatic carboxylic acid soluble salt forms (preferably acetate salt forms). The appropriate mga ratio (above indicated) of zirconium per gram mole of cobalt is likewise conveniently providable by the use of a combination of such carboxylic acid salts (acetates preferred) and bromide salts.

Suitable bromine sources include, for example, elemental bromine (Br₂), ionic bromide (for example, HBr, NaBr, KBr, and the like), or organic bromides, such as those which are known to provide bromide ions at the operating temperatures employed in the present liquid phase oxidation. Examples of such organic bromides include bromobenzenes, benzylbromide, mono-and di-bromo acetic acid, bromoacetyl bromide, tetrabromoethane, ethylene-di-bromide, and the like. The total bromine content (consisting of the total molecular bromine and ionic bromide) in the solvent medium is used to provide the desired ratio of elemental bromine to total cobalt and manganese in the mga ratio indicated above. The quantity of bromide ion released from the organic bromides at the oxidation operating conditions can be readily determined by known analytical means. For example, tetrabromoethane, at typical operating temperatures in the range from 172°C to 225°C (339°F to 437°F), has been found to yield in a reaction mixture such as above described about 3 gram atoms of bromine per gram mole of tetrabromoethane.

An illustrative and presently preferred resulting liquid effluent from the oxidation reaction zone comprises, on a 100 weight percent total basis, 15 to 25 weight percent IBPA, 75 to 85 weight percent total solvent comprised of water and aliphatic carboxylic acid (preferably acetic), 0 to 0.5 weight percent of unreacted DXP, 0.05 to 0.5 weight percent of oxidation catalyst components, 0.1 to 1 weight percent TMA, and from 0.2 to 2 weight percent of other reaction by-products. Other effluent compositions, however, can be achieved without departing from the spirit and scope of the process of this invention.

In one presently preferred mode of practicing this invention, a staged temperature/pressure profile is used in a single batch reaction zone to control the rate of reaction, and to permit a higher final temperature to be used, so as to maximize IBPA production while minimizing the production of unwanted by-products, such as TMA. Such a staged batch procedure is also desirable because it minimizes the need for adjustments in the air feed rate, such as would otherwise be necessary in order to maintain a chosen minimum oxygen gas concentration in the vent gas stream, such as a level within the range hereinabove indicated. Thus, a starting reactant mixture:
- having a solvent to DXP weight ratio of 4:1 to 8:1;
- where the solvent is comprised on a 100 weight percent total basis of 80 to 99.5 weight percent acetic acid, and, correspondingly, 20 to 0.5 weight percent water; and
- a zirconium-containing oxidation catalyst wherein the weight percent of cobalt dissolved in the solvent is in the range from 0.02 to 0.08; and the other catalyst components are present in amounts relative to the amount of cobalt present as hereinabove stated; is maintained at a temperature in the range of 154°C to 163°C (310°F to 325°F).

At this point, the first reaction stage commences as air is charged to the reactor (oxidation zone) at a feed rate which is conveniently 20 SCFH per pound of DXP. As the temperature increases to 166°C to 168°C, (330°F to 335°F) the air feed rate is increased to a rate of 80 to 85 SCFH per pound of DXP, within a total time interval of preferably not more than 2 to 3 minutes. A vigorous reaction is then characteristically observed to occur over about the next 10 minutes. The reaction rate slows slightly over about the next succeeding 5 minutes thereafter. During this 15 to 20 minute approximate total time period, reactor pressures are adjusted to maintain the temperature within the indicated range of 166°C to 168°C (330°F to 335°F). The oxygen level in the vent gas is maintained at 2 to 2.5 volume percent.

In a succeeding, or second, reaction phase, the reactor pressure is increased to elevate the reaction temperature to a range of 201°C to 205°C (395°F to 400°F) over a time period of from 20 to 25 minutes, a presently preferred temperature increase rate being 1.67°C per minute (3°F per minute). Typically, the reaction rate is maintained substantially constant for a time duration extending from the initiation of such second reaction phase for 15 to 30 minutes. During this period, the air feed rate is typically held constant in the range indicated with the vent oxygen level being maintained in the above indicated range of 2 to 2.5 volume percent, or 80 to 85 SCFH per pound of DXP.

However, during the next succeeding approximately 30 to 35 minute period, the reaction rate characteristically increases, and the air feed rate must then be increased to maintain the vent oxygen level from falling below 2 volume percent. During this particular period, the air feed rate is typically increased to a maximum 100 SCFH per pound of DXP.

Thereafter, the reaction rate starts to decrease inasmuch as a reactor pressure increase no longer increases the reaction zone temperature. The temperature may also begin to fall. At this point, the air feed rate is reduced to a level of 50 SCFH per pound of DXP. Such reduction is accomplished over a time interval of 2 to 5 minutes. This feed rate scale down is desirable because it decreases the amount of undesirable by-products, such as diacid phthalide (DAP) and TMA, and increases the yield of desired IBPA. Thereafter, pressure and temperature reductions down to ambient can be conventionally accomplished.

In general, a reaction product removed from the oxidation reaction zone is further processed using conventional techniques to separate and to recover a purified IBPA. Alternatively, the IBPA can be first dehydrated to the corresponding IPAN, and the IPAN recovered.

The present preferred dehydration process for converting crude IBPA to high purity IPAN utilizes the discovery that one solvent medium can serve multiple functions. Thus, as contemplated herein, an aromatic hydrocarbon solvent is not only a solvent for each of the starting IBPA and the product IPAN, but also is a constituent of a minimum-boiling azeotrope with water. Consequently, as a thermal, liquid phase dehydration of IBPA to IPAN is carried out to substantial completion, both starting material and product remain in solution, while concurrently the water produced by the dehydration is removed by azeotropic distillation using the same medium. Thereafter, purified IPAN is recovered from the same medium by merely cooling the product heated solution to cause precipitation of IPAN and then harvesting the precipitate. Thus, a simple, effective, and reliable technique is achieved for producing high purity IPAN directly from crude IBPA without problems caused by the solubility of IBPA in the oxidation reactor effluent from the oxidation of DXP. This technique avoids the purification problems experienced by the prior art and discussed hereinabove.

In the practice of the dehydration process of this invention, an IBPA-containing starting composition, preferably obtained from the hereinabove described oxidation process, is combined with an aromatic hydrocarbon which is a solvent for IPAN that also forms a minimum-boiling azeotrope with water to provide a dehydration composition. Preferably, the solvent is a lower alkylbenzene which is present in the dehydration composition in an amount which is at least sufficient to dissolve the IBPA present in the starting composition. Typical aromatic hydrocarbon solvents suitable for the present purpose include toluene, the xylenes and the trimethylbenzenes, especially pseudocumene (1,2,4-trimethylbenzene).

The dehydration composition comprising such aromatic hydrocarbon solvent and such starting composition is heated to a temperature in the range of 80°C to 220°C and is maintained at such a temperature while the water present, including the water formed by the resulting thermal dehydration of IBPA to the corresponding IPAN, is removed by azeotropic distillation. The azeotrope used is a binary water-xylene system. The temperature range employed is determined by the requirements for the dehydration step and by the temperature that is available for an azeotrope as produced in the process.

Next, the resulting IPAN-containing liquid residue is treated to separate therefrom insoluble impurities. This treatment can be carried out in any convenient manner, as desired, but preferably is accomplished while the liquid is at an elevated temperature immediately after the dehydration. A filtration medium, a centrifuge, or like liquid-solid separation means can be utilized. It is presently preferred to remove insoluble impurities by filtration using a filter aid, such as a diatomaceous earth-based filtering medium.

The resulting filtered dianhydride solution is then cooled to a temperature sufficient to induce precipitation of dissolved IPAN. It is presently preferred to cool the filtered solution to a temperature below 30°C, and more preferably to below 25°C, to precipitate IPAN therefrom.

The precipitated IPAN is separated from the produced mother liquor. As before, the liquid-solid separation can be accomplished by any convenient procedure, for example, by filtering at ambient conditions using a filter bed.

The recovered IPAN characteristically is at least 99 weight percent pure, contains less than 0.5 weight percent acid, contains relatively low levels of catalyst metal impurities, characteristically less than 200 ppm total, and contains relatively low levels of bromine (all forms), characteristically less than 200 ppm total. The product is well suited for use as a starting material for the production of polyimides.

There are several process variations which may be beneficial. For example, prior to the admixing of aromatic hydrocarbon solvent with the oxidation reactor effluent, the effluent can be heated to a temperature that is at least sufficient to remove by distillation a major portion of the water that is present as part of the oxidation solvent, thereby producing a liquid residue which is an IBPA-containing intractable oily substance. Typically, such a residue contains less than 5 weight percent water on a total residue weight basis. When, for example, the aliphatic carboxylic acid solvent is acetic acid, this oily substance is a mixture comprised of IBPA and acetic acid approximately in a 1:1 weight ratio. The aromatic hydrocarbon solvent above characterized is then added to this liquid residue, and the process step sequence is performed as above described.

If distillation of such a resulting oil is continued, almost all of the aliphatic carboxylic acid, such as acetic acid, can be removed from the oil and thus separated. However, when such a substantially aliphatic carboxylic acid-free oil is then processed as contemplated by this invention, the IPAN product obtained has a more yellow color than when the starting material is a crude IBPA composition as above characterized, or when the oil to be processed contains a mixture of IBPA and the aliphatic carboxylic acid.

Also, activated carbon can be added to the admixture of the oxidation reactor effluent (or of the intractable oil product) with the aromatic hydrocarbon solvent before, or during, the azeotropic distillation. The amount of activated carbon so added conveniently and preferably can be in the range of about 1 to about 5 percent, based upon IBPA content, though larger and smaller amounts may be employed, if desired. The added activated carbon may improve final product purity, and increase the rate of dehydration as well.

If a still higher purity IPAN product is desired, an additional purification step or steps can be performed. For example, the recovered IPAN product can be redissolved in the aromatic hydrocarbon solvent and then recrystallized using an additional heating and cooling cycle such as above described.

The following examples are offered to specifically illustrate this invention. These examples are not to be construed as limiting the scope thereof, however.

### Example 1: Effect of Zr/Co and Br/(Co+Mn) Ratio

To an agitator- and condenser-equipped pressurizable reactor vessel are charged batchwise:

**TABLE I**

| Starting Material Composition Initially Charged to Reactor | |
|---|---|
| Component | Weight Percent (100 weight percent total composition weight basis) |
| DXP | 13.0 |
| Acetic Acid | 82.4 |
| Water | 4.4 |
| Oxidation Catalyst | 0.2 |

The concentration of the cobalt catalyst component in the starting reactant mixture composition relative to DXP was about 6 milligram atoms of cobalt per gram mole of DXP, and the weight percent of cobalt based on 100 weight percent combined weight of total solvent plus cobalt was about 0.02 weight percent. The starting composition contained also about 1 milligram atom of manganese per milligram atom of cobalt, no zirconium, and bromine in a bromine to total cobalt-and-manganese mole ratio of about 1. The catalyst had the composition shown in Table II, below:

**TABLE II**

| Oxidation Catalyst Composition | |
|---|---|
| Component | Weight Percent (100 weight percent total catalyst weight basis) |
| Cobalt (acetate tetrahydrate) | 30.2 |
| Manganese (acetate tetrahydrate) | 30.2 |
| Bromine (HBr) (as 48% HBr in water) | 39.6 |
| Total Bromine | 22.3 |

This catalyst composition was preliminarily dissolved in a portion of the oxidation solvent, i.e., acetic acid and water, which was charged to the reactor initially. After the charging, pressurized air was continuously fed to the reactor at a rate of about 0.2 SCFM while the reactor was maintained at a temperature of about 177°C and at an autogenous pressure corresponding to such temperature. The batch oxidation was continued for about 80 minutes.

Analysis of the batch liquid reaction product showed that the yield of IBPA was about 5.1 grams (equal to a conversion efficiency of about 5.7 mole percent based on starting DXP) while the yield of TMA was about 0.4 grams (equal to about 0.7 mole percent based on starting DXP).

The catalyst amounts, temperature, and results are summarized below in Table III, below, following Examples 2-4.

### Examples 2-4: Effect of Zr/Co and Br/(Co+Mn) Ratios (Continued)

The procedure of Example 1 was repeated a plurality of times, except that in each replication a different catalyst amount, and a different mole ratio of cobalt, manganese, zirconium, and bromine, were employed.

Table III below summarizes catalyst amounts as weight percent cobalt content based on solvent and as mole ratios of catalyst components expressed as Co:Mn:Zr:Br used in each replication. To achieve these respective mole ratios, the composition of the starting catalyst was varied from the catalyst composition shown above in Table II to provide the indicated catalyst component proportions. The reactor temperature, and the reaction yields in mole percent, are also shown in Table III.

The present Examples 1 to 4 illustrate the effects of changes in the cobalt/zirconium ratio and in the Br/(Co+Mn) ratio. In each pair of runs, identified in Table III as Example 1 and Example 2, and as Example 3 and Example 4, one run (Example 1 and Example 3, respectively) was made without the use of zirconium, and the other run (Example 2 and Example 4, respectively) with the use of zirconium. In each run where the zirconium was present, it was found that the addition of only a small amount of zirconium produces a tremendous and surprising increase in the IBPA yield. The effect is more pronounced in the Example 1/Example 2 pair because a lower overall catalyst loading was used.

Comparison of Example 2 with Example 4 provides an insight into the effect of the Br/(Co+Mn) ratio. Thus, in Example 4, higher temperatures and higher metals loadings were used than in Example 2. The yield value achieved in Example 2 is believed to be typical for a Mid-Century type oxidation process procedure as practiced in the prior art. These conditions were expected, based on prior art experience, to lead to the formation of more TMA. However, in Example 4, the TMA yield is unexpectedly much lower than that in Example 2. It is believed that this is because of the very low molar Br/(Co+Mn) ratio of 0.1 used in Example 4, as opposed to the ratio of 1.0 used in Example 2.

**TABLE III**

| Effects of Varying Catalyst Concentration and Components | | | | |
|---|---|---|---|---|
| Variable | Ex. 1 | Ex. 2 | Ex. 3 | Ex. 4 |
| Temperature °F. (°C) | 350 (177) | 350 (177) | 385 (196) | 385 (196) |
| Wt% Co on total solvent | 0.02 | 0.02 | 0.08 | 0.08 |
| Co:Mn:Zr:Br (molar ratio) | 1:1:0:2 | 1:1:0.1:2 | 1:2:0:1 | 1:2:0.1:0.3 |
| Solvent: DXP weight ratio | 7:1 | 7:1 | 7:1 | 7:1 |
| Reaction Yields mole % IBPA | 5.7 | 82.8 | 66.1 | 86.9 |
| TMA | 0.7 | 7.3 | 2.4 | 3.7 |

### Examples 5-7: Temperature Effect on Oxidation

The procedure of Example 1 was repeated except that different catalyst amounts and different mole ratios of cobalt, manganese, zirconium, and bromine were employed in each of various starting mixtures. TABLE IV, below, summarizes the catalyst amounts (expressed as weight percent of cobalt content based on total amount of solvent and cobalt weight) and mole ratios of catalyst components (expressed as Co:Mn:Zr:Br). To achieve these respective mole ratios for each Example, the composition of the starting catalyst was adjusted from the composition shown in Example 1.

As TABLE IV below shows, the amount of TMA produced increases as the reaction temperature increases.

**TABLE IV**

| Effect of Temperature in Batch DXP Oxidations | | | |
|---|---|---|---|
| | Run | | |
| Variable | A | B | C |
| Temperature °F. (°C) | 385 (196) | 350 (177) | 310 (149) |
| Wt% Co on total solvant | 0.08 | 0.08 | 0.08 |
| Co:Mn:Zr:Br (molar ratio) | 1:2:0.1:0.3 | l:2:0.1:0.3 | 1:2:0.1:0.3 |
| Solvent:DXP (weight ratio) | 7:1 | 7:1 | 7:1 |
| Run Time (min.) | 31 | 31 | 52 |
| Reactor Yields (mole %) IBPA | 85.3 | 86.3 | 75.6 |
| TMA | 3.7 | 2.3 | 1.1 |

### Example 8: Evaluation

The procedure of Example 1 was repeated except that the reactor temperature employed was 350°F. (171°C), the weight percent of cobalt based on solvent was 0.08, the molar ratio of Co:Mn:Zr:Br was 1:2:0.1:0.3, and the solvent/DXP weight ratio was 7:1. Using these conditions, a set of 20 batch oxidations of DXP to IBPA were performed in five groups consisting of an average of four runs each.

The results are summarized in TABLE V, below. Specifically, TABLE V contains the results from each of the five groups, along with the overall mean and standard deviation for each.

These results confirm that a batch oxidation process of the present invention employing a catalyst of zirconium, cobalt, manganese and bromine and utilizing a relatively low temperature and a relatively low Br/(Co+Mn) mole ratio produces a relatively high yield of IBPA with only a relatively small amount of TMA.

**TABLE V**

| Results from Multiple IBPA Production Batches | | | | | | |
|---|---|---|---|---|---|---|
| Product Component | Yield for Run No., mol-% | | | | | Mean ± Std. Dev. for All Runs |
| | 1 | 2 | 3 | 4 | 5 | |
| IBPA | 93.5 | 91.8 | 86.5 | 86.1 | 86.6 | 88.9 ± 3.5 |
| TMA | 3.0 | 3.0 | 3.3 | 3.3 | 3.7 | 3.3 ± 0.3 |

### Example 9: Staged Batch Process

DXP was oxidized to IBPA in a batch reactor using a solvent-to-DXP weight ratio of about 6.75:1 where the solvent initially comprised, on a 100 weight percent solvent basis, about 95 weight percent acetic acid and about 5 weight percent water.

The solvent contained about 0.03 weight percent of dissolved cobalt (calculated as elemental cobalt), and the molar ratio of dissolved catalyst components with respect to cobalt, expressed as Co:Mn:Zr:Br, was 1.:2.:0.06:0.3. The catalyst concentration was relatively low in order to minimize the initial reaction rate. A very low molar Br/(Co + Mn) ratio of about 0.1 was used to minimize TMA formation and to slow down the initial rate of reaction. The presence of a small amount of zirconium minimized the amount of oxidation intermediates formed and drove the reaction to completion.

In a first reaction stage, the DXP, solvent and catalyst were heated with agitation to about 320°F (about 160°C) and air was then charged continuously commencing at a rate of about 20 SCFH per pound of DXP. The temperature was increased over a time period of about 3 minutes to about 330°F (about 166°C) and held at this level for about 15 minutes. As the temperature was thus being increased, the air feed rate was increased to about 83 SCFH per pound of DXP and was held at this level. Over about the first 10 minutes, the reaction was observed to be very vigorous, after which time interval the reaction rate slowed slightly over the next approximately 5-minute period. Throughout this time period, the reactor pressure was adjusted to maintain 330°F (166°C). The vent O₂ level was in the range of 2 to 2.5 volume percent.

In the second reaction stage, which began at about 15 minutes after oxidation was initiated, the pressure was ramped (continuously increased) to elevate the reaction temperature from 330°F (166°C) to 395°F to 400°F (201°C to 204°C) over a time period of about 22 minutes at the approximate rate of 3°F/minute (1.67°C/min.). A temperature in this range served to hold the reaction rate substantially constant during the next time period of 15 to 30 minutes after its initiation. During this period the air feed rate was held constant with the vent O₂ level being in the range indicated. However, during the next approximately 30 to 35 minute time period thereafter, the reaction rate increased, and the air feed rate was increased to a maximum of about 100 SCFH per pound of DXP to prevent the vent gas O₂ level from falling below 2 percent.

The start of the next or third reaction stage was marked by the point at which the reaction rate decreased as shown by the fact that upon increasing the reactor pressure the reaction temperature did not increase. At this point, the air feed rate was reduced over about a 2 to 5 minute period. Such action decreased the quantity of the unwanted by-product diacid phthalide (DAP) intermediate produced and also of unwanted TMA produced. Thereafter, heating was terminated, air feed was halted, and the reactor was depressurized. Optionally, solvent withdrawal by distillation can be used to reduce to weight of the total reactor effluent.

The IBPA yield was 89 to 90 mole percent, TMA yield was 4.5 to 5.5 mole percent, and the maximum DAP yield was 0.8 to 1 percent that of IBPA.

## Claims

1. A method of producing isopropylidene bis(phthalic acid) which comprises oxidising dixylylpropane with a source of molecular oxygen in the liquid phase in the presence of a catalyst and an aqueous oxidation solvent comprising an aliphatic acid having 2 to 6 carbon atoms, characterised in that the catalyst comprises sources of zirconium, cobalt, manganese and bromine.

2. A method according to claim 1 wherein the catalyst comprises cobalt, manganese, zirconium and bromine components which are each dissolved in said solvent.

3. A method according to Claim 1 or Claim 2 wherein there are present:
- 1 to 50 milligram atoms of cobalt per gram mole of said dixylylpropane,
- 0.1 to 10 milligram atoms of manganese per milligram atom of cobalt, and
- 0.01 to 1 milligram atoms of zirconium per milligram atom of cobalt.

4. A method according to any preceding claim wherein the catalyst comprises
- 0.05 to 2.5 milligram atoms of bromine calculated as elemental bromine dissolved in said solvent per milligram atom of said cobalt.

5. A method according to any preceding claim wherein the bromine-to-(cobalt plus manganese) mole ratio is 0.02 to 1.

6. A method according to any preceding claim wherein the weight ratio of said solvent to said dixylylpropane is in the range of
2:1 to 10:1.

7. A method according to Claim 6 wherein the solvent-to-dixylylpropane weight ratio is 2.5:1 to 8:1.

8. A method according to any preceding claim wherein the reaction is maintained until the yield of said isopropylidene bis(phthalic acid) is greater than 85 mole percent based on starting dixylylpropane.

9. A method according to any preceding claim wherein said aliphatic carboxylic acid is acetic acid.

10. A method according to any preceding claim wherein said oxygen is supplied to said reaction zone as air, and, during said oxidation, said air is continuously supplied to said reaction zone at a feed rate which is sufficient to provide in said reaction zone oxygen in an amount of 1.6 to 2.8 moles per methyl group of said dixylylpropane present.

11. A method according to any preceding claim wherein a molar excess of oxygen relative to dixylylpropane is maintained.

12. A method according to any preceding claim wherein said oxidation is effected at a temperature in the range 100°C to 240°C and at an elevated pressure.

13. A method according to any preceding claim comprising the steps of:
- introducing into an oxidation reactor dixylylpropane, an oxidation solvent containing an aliphatic acid having 2 to 6 carbon atoms per molecule, an oxygen-containing gas, and a zirconium-containing oxidation catalyst system soluble in said oxidation solvent and additionally constituted by cobalt, manganese, and bromine;
- agitating the resulting reactor contents to produce an admixture;
- maintaining the produced admixture in said reactor at a temperature in the range of 100°C to 240°C and at autogenous elevated pressure for a time period to produce a reaction mixture enriched in isopropylidene bis(phthalic acid); and
- withdrawing from said reactor an effluent stream having a relatively lower dixylylpropane content than said admixture and containing isopropylidene bis(phthalic acid).

14. A method according to any preceding claim comprising the steps of
(A) charging to a reaction zone each of
(1) dixylylpropane,
(2) solvent comprising on a 100 weight percent basis 0.5 to 20 weight percent of water and 80 to 99.5 weight percent of at least one aliphatic carboxylic acid having from 2 to 6 carbon atoms per molecule, the weight ratio of said solvent to said dixylylpropane being in the range from 2:1 to 10:1,
(3) oxygen in an amount sufficient to produce a molar excess of oxygen to said dixylylpropane, and
(4) an oxidation catalyst comprising cobalt, manganese, zirconium, and bromine components which are each dissolved in said solvent,
(B) maintaining the resulting mixture in said reaction zone under autogeneous pressure and at a temperature in the range of 100°C to 240°C for a time period sufficient to oxidize at least 75 mole percent of the charged dixylylpropane while agitating said mixture, and
(C) removing from said reaction zone the resulting product mixture.

15. A method according to Claim 14 wherein said steps are practised batchwise.

16. A method according to Claim 14 wherein said steps are practised continuously.

17. A catalyst system suitable for liquid phase oxidation of dixylylpropane to isopropylidene bis(phthalic acid) which comprises a solution of ionic forms of zirconium, cobalt, manganese, and bromine in an aqueous oxidation solvent containing aliphatic acid having 2 to 6 carbon atoms.

18. The catalyst system of claim 17 wherein:
- the cobalt component, calculated as elemental cobalt, is present in said solution at a level of 0.05 to 3 milligram atoms of cobalt per each 100 parts by weight of said solution,
- the manganese component, calculated as elemental manganese, is present in said solution at a level of 0.1 to 10 milligram atoms of manganese per milligram atom of said cobalt,
- the zirconium component, calculated as elemental zirconium, is present in said solution at a level of 0.01 to 1 milligram atoms of zirconium per milligram atom of said cobalt,
- the bromine component, calculated as elemental bromine, is present in said solution at a respective bromine-to-(cobalt plus manganese) mole ratio of 0.02 to 1, and
- said solution on a 100 weight percent total solution weight basis is comprised of 80 to 99.5 weight percent of said aliphatic acid, and, correspondingly, 0.5 to 20 weight percent of water.

## Patentansprüche

1. Verfahren zur Herstellung von Isopropyliden-bis(phthalsäure), welches umfaßt: Oxidieren von Dixylylpropan mit einer Quelle molekularen Sauerstoffs in der flüssigen Phase in Gegenwart eines Katalysators und eines wäßrigen Oxidationslösemittels, welches eine aliphatische Säure mit 2 bis 6 Kohlenstoffatomen umfaßt, dadurch gekennzeichnet, daß der Katalysator Quellen von Zirkon, Kobalt, Mangan und Brom umfaßt.

2. Verfahren gemäß Anspruch 1, worin der Katalysator Kobalt-, Mangan-, Zirkon- und Brom-Komponenten umfaßt, die jeweils in dem Lösemittel gelöst sind.

3. Verfahren gemäß Anspruch 1 oder Anspruch 2, worin vorhanden sind:
- 1 bis 50 Milligramm-Atom Kobalt pro Gramm-Mol Dixylylpropan,
- 0.1 bis 10 Milligramm-Atom Mangan pro Milligramm-Atom Kobalt und
- 0.01 bis 1 Milligramm-Atom Zirkon pro Milligramm-Atom Kobalt.

4. Verfahren gemäß irgendeinem der vorhergehenden Ansprüche, worin der Katalysator umfaßt:
- 0.05 bis 2.5 Milligramm-Atom Brom, berechnet als elementares Brom, gelöst im Lösemittel, pro Milligramm-Atom Kobalt.

5. Verfahren gemäß irgendeinem der vorhergehenden Ansprüche, worin das Molverhältnis von Brom zu (Kobalt plus Mangan) 0.02 bis 1 ist.

6. Verfahren gemäß irgendeinem der vorhergehenden Ansprüche, worin das Gewichtsverhältnis von Lösemittel zu Dixylylpropan im Bereich von 2 : 1 bis 10 : 1 liegt.

7. Verfahren gemäß Anspruch 6, worin das Gewichtsverhältnis Lösemittel zu Dixylylpropan 2.5 : 1 bis 8 : 1 beträgt.

8. Verfahren gemäß irgendeinem der vorhergehenden Ansprüche, worin die Reaktion aufrechterhalten wird, bis die Ausbeute an Isopropyliden-bis(phthalsäure) größer als 85 Mol-%, bezogen auf das Ausgangs-Dixylylpropan, ist.

9. Verfahren gemäß irgendeinem der vorhergehenden Ansprüche, worin die aliphatische Carbonsäure Essigsäure ist.

10. Verfahren gemäß irgendeinem der vorhergehenden Ansprüche, worin der Sauerstoff der Reaktionszone als Luft zugeführt wird und die Luft während der Oxidation der Reaktionszone kontinuierlich zugeführt wird mit einer Zufuhrrate, die ausreichend ist, um in der Reaktionszone Sauerstoff in einer Menge von 1.6 bis 2.8 Mol pro Methylgruppe des vorhandenen Dixylylpropans zur Verfügung zu stellen.

11. Verfahren gemäß irgendeinem der vorhergehenden Ansprüche, worin ein molarer Überschuß an Sauerstoff relativ zum Dixylylpropan aufrechterhalten wird.

12. Verfahren gemäß irgendeinem der vorhergehenden Ansprüche, worin die Oxidation bei einer Temperatur im Bereich von 100 bis 240 °C und bei erhöhtem Druck bewirkt wird.

13. Verfahren gemäß irgendeinem der vorhergehenden Ansprüche, welches die Schritte umfaßt:
- Einführen in einen Oxidationsreaktor von Dixylylpropan, einem Oxidationslösemittel, das eine aliphatische Säure mit 2 bis 6 Kohlenstoffatomen pro Molekül enthält, einem sauerstoffhaltigen Gas und einem zirkonhaltigen Oxidationskatalysator-System, das in dem Oxidationslösemittellöslich ist und zusätzlich aus Kobalt, Mangan und Brom gebildet wird,
- Rühren des resultierenden Reaktorinhalts, um eine Mischung zu erzeugen,
- Halten der erzeugten Mischung in dem Reaktor bei einer Temperatur im Bereich von 100 °C bis 240 °C und bei autogen erhöhtem Druck eine hinreichende Zeit lang, um eine Reaktionsmischung zu ergeben, die an Isopropyliden-bis(phthalsäure) angereichert ist, und
- Abziehen eines Ausstroms, der einen relativ geringeren Dixylylpropan-Gehalt besitzt als die Mischung und der Isopropyliden-bis(phthalsäure) enthält, aus dem Reaktor.

14. Verfahren gemäß irgendeinem der vorhergehenden Ansprüche, welches die Schritte umfaßt:
(A) Beschicken einer Reaktionszone jeweils mit
(1) Dixylylpropan,
(2) Lösemittel, welches, auf einer 100 Gew.-%-Basis, 0.5 bis 20 Gew.-% Wasser und 80 bis 99.5 Gew.-% mindestens einer aliphatischen Carbonsäure mit 2 bis 6 Kohlenstoffatomen pro Molekül enthält, wobei das Gewichtsverhältnis des Lösemittels zum Dixylylpropan im Bereich von 2 : 1 bis 10 : 1 liegt,
(3) Sauerstoff in hinreichender Menge, um einen molaren Überschuß an Sauerstoff zum Dixylylpropan zu erzeugen, und
(4) einen Oxidationskatalysator, der Kobalt-, Mangan-, Zirkon- und Brom-Komponenten umfaßt, die jeweils in dem Lösemittel gelöst sind,
(B) Halten der resultierenden Mischung in der Reaktionszone unter autogenem Druck und bei einer Temperatur im Bereich von 100 °C bis 240 °C eine hinreichende Zeit lang, um mindestens 75 Mol-% des zugeführten Dixylylpropans zu oxidieren, wobei die Mischung gerührt wird, und
(C) Entfernen der resultierenden Produktmischung aus der Reaktionszone.

15. Verfahren gemäß Anspruch 14, worin die Schritte batchweise durchgeführt werden.

16. Verfahren gemäß anspruch 14, worin die Schritte kontinuierlich durchgeführt werden.

17. Katalysatorsystem, geeignet für die Oxidation von Dixylylpropan zu Isopropyliden-bis(phthalsäure) in der flüssigen Phase, welches eine Lösung ionischer Formen von Zirkon, Kobalt, Mangan und Brom in einem wäßrigen Oxidationslösemittel, das eine aliphatische Säure mit 2 bis 6 Kohlenstoffatomen enthält, umfaßt.

18. Katalysatorsystem gemäß Anspruch 17, worin
- die Kobaltkomponente, berechnet als elementares Kobalt, in der Lösung in einer Menge von 0.05 bis 3 Milligramm-Atom Kobalt pro jeweils 100 Gew.-Teile der Lösung vorhanden ist,
- die Mangankomponente, berechnet als elementares Mangan, in der Lösung in einer Menge von 0.1 bis 10 Milligramm-Atom Mangan pro Milligramm-Atom Kobalt vorhanden ist,
- die Zirkonkomponente, berechnet als elementares Zirkon, in der Lösung in einer Menge von 0.01 bis 1 Milligramm-Atom Zirkon pro Milligramm-Atom Kobalt vorhanden ist,
- die Bromkomponente, berechnet als elementares Brom, in der Lösung in einem entsprechenden Molverhältnis von Brom zu (Kobalt plus Mangan) von 0.02 bis 1 vorhanden ist, und
- die Lösung, auf einer Basis von 100 Gew.-% der gesamten Lösung, 80 bis 99.5 Gew.-% aliphatische Säure und, entsprechend, 0.5 bis 20 Gew.-% Wasser umfaßt.

## Revendications

1. Procédé de production d'acide isopropylidène bis-phtalique, qui comprend l'oxydation de dixylylpropane avec une source d'oxygène moléculaire en phase liquide, en présence d'un catalyseur et d'un solvant d'oxydation aqueux comprenant un acide aliphatique comportant 2 à 6 atomes de carbone, caractérisé en ce que le catalyseur comprend des sources de zirconium, de cobalt, de manganèse et de brome.

2. Procédé selon la revendication 1, dans lequel le catalyseur comprend des constituants du cobalt, du manganèse, du zirconium et du brome qui sont chacun dissous dans ledit solvant.

3. Procédé selon la revendication 1 ou 2, dans lequel sont présents:
- 1 à 50 milliatome-grammes de cobalt par mole-gramme dudit dixylylpropane,
- 0,1 à 10 milliatome-grammes de manganèse par milliatome-gramme de cobalt, et
- 0,01 à 1 milliatome-gramme de zirconium par milliatome-gramme de cobalt.

4. Procédé selon l'une quelconque des revendications précédentes, dans lequel le catalyseur comprend
- 0,05 à 2,5 milliatome-grammes de brome, calculé en brome élémentaire, dissous dans ledit solvant, par milliatome-gramme dudit cobalt.

5. Procédé selon l'une quelconque des revendications précédentes, dans lequel le rapport molaire brome/(cobalt plus manganèse) est de 0,02 à 1.

6. Procédé selon l'une quelconque des revendications précédentes, dans lequel le rapport pondéral dudit solvant audit dixylylpropane est dans la gamme de 2:1 à 10:1.

7. Procédé selon la revendication 6, dans lequel le rapport pondéral solvant/dixylylpropane est de 2,5:1 à 8:1.

8. Procédé selon l'une quelconque des revendications précédentes, dans lequel la réaction est poursuivie jusqu'à ce que le rendement dudit acide isopropylidène bis-phtalique soit supérieur à 85% en moles, par rapport au dixylylpropane de départ.

9. Procédé selon l'une quelconque des revendications précédentes, dans lequel ledit acide carboxylique aliphatique est l'acide acétique.

10. Procédé selon l'une quelconque des revendications précédentes, dans lequel ledit oxygène est amené dans ladite zone réactionnelle sous forme d'air et, au cours de ladite oxydation, ledit air est amené en continu dans ladite zone réactionnelle, à un débit d'alimentation qui est suffisant pour fournir à ladite zone réactionnelle de l'oxygène en une quantité de 1,6 à 2,8 moles par groupe méthyle dudit dixylylpropane présent.

11. Procédé selon l'une quelconque des revendications précédentes, dans lequel on maintient un excès molaire de l'oxygène par rapport au dixylylpropane.

12. Procédé selon l'une quelconque des revendications précédentes, dans lequel ladite oxydation est réalisée à une température dans la gamme de 100°C à 240°C et sous une pression élevée.

13. Procédé selon l'une quelconque des revendications précédentes, comprenant les étapes qui consistent à:
- introduire, dans un réacteur d'oxydation, du dixylylpropane, un solvant d'oxydation contenant un acide aliphatique comportant 2 à 6 atomes de carbone par molécule, un gaz contenant de l'oxygène et un système catalytique d'oxydation contenant du zirconium, soluble dans ledit solvant d'oxydation et constitué en sus de cobalt, de manganèse et de brome;
- agiter le contenu du réacteur obtenu pour produire un mélange;
- maintenir le mélange produit dans ledit réacteur, à une température dans la gamme de 100°C à 240°C et sous la pression autogène élevée, pendant une durée permettant de produire un mélange réactionnel enrichi en acide isopropylidène bis-phtalique, et
- soutirer dudit réacteur un courant d'effluent ayant une teneur en dixylylpropane relativement plus faible que ledit mélange et contenant de l'acide isopropylidène bis-phtalique.

14. Procédé selon l'une quelconque des revendications précédentes, comprenant les étapes qui consistent à
(A) charger dans une zone réactionnelle
(1) du dixylylpropane,
(2) un solvant comprenant, rapporté à 100% en poids, 0,5 à 20% en poids d'eau et 80 à 99,5% en poids d'au moins un acide carboxylique aliphatique comportant 2 à 6 atomes de carbone par molécule, le rapport pondéral dudit solvant audit dixylylpropane étant dans la gamme de 2:1 à 10:1,
(3) de l'oxygène, en quantité suffisante pour produire un excès molaire d'oxygène par rapport audit dixylylpropane, et
(4) un catalyseur d'oxydation comprenant des constituants cobalt, manganèse, zirconium et brome, qui sont chacun dissous dans ledit solvant,
(B) maintenir le mélange résultant dans ladite zone réactionnelle sous la pression autogène et à une température dans la gamme de 100°C à 240°C, pendant suffisamment longtemps pour oxyder au moins 75% en moles du dixylylpropane chargé, tout en agitant ledit mélange, et
(C) retirer de ladite zone réactionnelle le mélange de produit résultant.

15. Procédé selon la revendication 14, dans lequel lesdites étapes sont réalisées en discontinu.

16. Procédé selon la revendication 14, dans lequel lesdites étapes sont réalisées en continu.

17. Système catalytique convenant à une oxydation en phase liquide de dixylylpropane en acide isopropylidène bis-phtalique, qui comprend une solution de formes ioniques du zirconium, du cobalt, du manganèse et du brome, dans un solvant d'oxydation aqueux contenant un acide aliphatique comportant 2 à 6 atomes de carbone.

18. Système catalytique selon la revendication 17, dans lequel:
- le constituant cobalt, calculé en cobalt élémentaire, est présent dans ladite solution à raison de 0,05 à 3 milliatome-grammes de cobalt pour 100 parties en poids de ladite solution,
- le constituant manganèse, calculé en manganèse élémentaire, est présent dans ladite solution à raison de 0,1 à 10 milliatome-grammes de manganèse par milliatome-gramme dudit cobalt,
- le constituant zirconium, calculé en zirconium élémentaire, est présent dans ladite solution à raison de 0,01 à 1 milliatome-gramme de zirconium par milliatome-gramme dudit cobalt,
- le constituant brome, calculé en brome élémentaire, est présent dans ladite solution dans un rapport molaire respectif brome/(cobalt plus manganèse) de 0,02 à 1, et
- ladite solution, pour 100% en poids de solution totale, est constituée de 80 à 99,5% en poids dudit acide aliphatique et, par conséquent, de 0,5 à 20% en poids d'eau.
